# EUROPEAN PATENT SPECIFICATION

(11) **EP 3 918 055 B1**
(45) Date of publication and mention of the grant of the patent: **27.08.2025**
(21) Application number: 20749209.1
(22) Date of filing: 22.01.2020
(51) Int. Cl.: A61K 35/744, A61K 35/747, A61P 25/00

(54) **METHOD FOR PREVENTING OR TREATING ABNORMAL EMOTION OR BEHAVIOR IN NON-HUMAN ANIMAL BY LACTIC ACID BACTERIUM**
VERFAHREN ZUR VORBEUGUNG ODER BEHANDLUNG VON ANORMALEM GEFÜHL ODER VERHALTEN BEI EINEM NICHT-MENSCHLICHEN TIER DURCH MILCHSÄUREBAKTERIEN
PROCÉDÉ DE PRÉVENTION OU DE TRAITEMENT D'UNE ÉMOTION ANORMALE OU D'UN COMPORTEMENT ANORMAL CHEZ UN ANIMAL NON HUMAIN PAR BACTÉRIE LACTIQUE

(30) Priority: 01.02.2019 US 201962800150 P
(43) Date of publication of application: 08.12.2021
(73) Proprietor: Bened Biomedical Co., Ltd., Taipei City 104 (TW)
(72) Inventor: TSAI, Ying-Chieh, Zhongshan Dist. Taipei City, Taiwan (CN); WANG, Lih-Chiann, Zhongshan Dist. Taipei City, Taiwan (CN); HUANG, Chin-Lin, Zhongshan Dist., Taipei City, Taiwan (CN); WU, Chien-Chen, Zhongshan Dist., Taipei City, Taiwan (CN); HSU, Chih-Chieh, Zhongshan Dist., Taipei City, Taiwan (CN)
(74) Representative: Rösler Rasch van der Heide & Partner
(86) International application number: PCT/CN2020/073755
(87) International publication number: WO 2020/156418

(56) References cited:
- EP-A1- 2 937 424
- WO-A1-2018/014225
- TW-A- 201 811 346
- US-A1- 2015 306 157
- DAVIS DANIEL J ET AL: "Lactobacillus plantarum attenuates anxiety-related behavior and protects against stress-induced dysbiosis in adult zebrafish", SCIENTIFIC REPORTS, NATURE PUBLISHING GROUP, US, vol. 6, 19 September 2016 (2016-09-19), XP009511036, ISSN: 2045-2322, DOI: 10.1038/SREP33726
- LIU YEN-WENN; LIU WEI-HSIEN; WU CHIEN-CHEN; JUAN YI-CHEN; WU YU-CHEN; TSAI HUEI-PING; WANG SABRINA; TSAI YING-CHIEH: "Psychotropic effects of Lactobacillus plantarum PS128 in early life-stressed and naïve adult mice.", BRAIN RESEARCH, no. 1631, 15 January 2016 (2016-01-15), XP029396287, ISSN: 0006-8993, DOI: 10.1016/j.brainres.2015.11.018
- LIU WEI-HSIEN; CHUANG HSIAO-LI; HUANG YEN-TE; WU CHIEN-CHEN; CHOU GENG-TING; WANG SABRINA; TSAI YING-CHIEH: "Alteration of behavior and monoamine levels attributable to Lactobacillus plantarum PS128 in germ-free mice", BEHAVIOURAL BRAIN RESEARCH, vol. 298, no. 298, 29 October 2015 (2015-10-29), pages 202 - 209, XP029343593, ISSN: 0166-4328, DOI: 10.1016/j.bbr.2015.10.046
- IAO JIAN-FU; CHENG YUN-FANG; LI SHIAO-WEN; LEE WANG-TSO; HSU CHIH-CHIEH; WU CHIEN-CHEN; JENG ONE-JANG; WANG SABRINA; TSAI YING-CHI: "Lactobacillus plantarum PS128 ameliorates 2, 5-Dimethoxy-4-iodoamphetamine-induced tic-like behaviors via its influences on the microbiota-gut-brain-axis.", BRAIN RESEARCH BULLETIN, no. 153, 25 July 2019 (2019-07-25), pages 59 - 73, XP085891415, ISSN: 0361-9230, DOI: :10.1016/j.brainresbull.2019.07.027

## Description

### BACKGROUND

### 1. Technical Field

This disclosure relates to methods for preventing or treating emotional and behavioral problems in animals, and relates particularly to methods for preventing or treating aggression in non-human animals,

### 2. Description of Related Art

The health benefits of companion or domestic animals have been widely recognized in the public. Research shows that pet owners have less illness, recover faster from serious health conditions, and tend to be more content than people who do not own pets. Some specific potential benefits of owning companion animals include fewer feelings of loneliness, more compatibility among humans, fewer allergies, lower blood pressure, and enhanced ability to meet others and be more social, which are strongly associated with good mental and physical health.

However, just like human and other animals, companion and domestic animals can develop emotional and behavioral problems as well. These problems could weaken the pet-owner bond, resulting in a decreased owner commitment to pet care. In fact, the emotional and behavioral problems are a primary reason for pet relinquishment to a humane society or shelter, abandonment, or euthanasia. For example, one frequently cited reason for returning a dog is behavior attributed to separation anxiety. It has been estimated that in the average veterinary practice in the United States, up to 14% of canine patients exhibit one or more signs of separation anxiety, including abnormal elimination, destruction, and vocalization. Other behaviors associated with anxiety may include salivation, anorexia, and lethargy.

Thus, when these problems develop in companion and domestic animals, they require immediate attention and treatment. In companion animals, the treatment of behavioral problems varies with diagnosis and prognosis. In general, the treatment program begins with behavior modification that starts with prevention and avoidance of problems. Initially, prevention is necessary to avoid further compromising the pet's welfare and to ensure safety in cases of aggression. At the same time, the owner needs to develop effective strategies to modify the pet's behavior so that it might gradually be reintroduced to the problem situations while achieving a desirable outcome. Repetition of the behavior further aggravates the problem if the pet successfully accomplishes its intended goal (e.g., escape or retreat from the stimulus), while each exposure in which the outcome is unpleasant can condition further anxiety. Improvement is generally a slow and gradual process, and modifications to the environment may be required, so that the pet can be kept away from the stimuli (or the sights or sounds of the stimuli). Modifying the pet's behavior is accomplished by applying the principles of learning and behavior modification, primarily achieving and rewarding desirable outcomes along with use of products that improve safety, reduce anxiety, or help to achieve the desired response more effectively (e.g., muzzles, head halters, no-pull harnesses, etc.).

Drugs and natural products may also be indicated for some pets and some problems. A variety of natural products have been used to treat anxiety; however, only a few have demonstrated evidence to support efficacy. There has been recommendation on the psychoactive drugs as adjuncts to behavior modification in the treatment of selected cases of nuisance barking, such as amitriptyline, buspirone, clomipramine, and fluoxetine.

Accordingly, there remains an unmet need to provide an effective prevention or treatment for abnormal emotions and behaviors in non-human animals.

### SUMMARY

In this disclosure, it is found that a lactic acid bacterium, *Lactobacillus plantarum* subsp. *plantarum* PS128 (hereinafter referred to as PS128), is effective in preventing or treating an abnormal emotion or behavior in a non-human animal, e.g., for preventing or treating aggression. In one embodiment, a composition for use in preventing or treating an abnormal emotion or behavior in a non-human animal, characterized in that the composition comprises an effective amount of Lactobacillus plantarum subsp. plantarum PS128, which is deposited under DSMZ Accession No. DSM 28632, and a carrier thereof, wherein the composition improves a score or a value for evaluating the abnormal emotion or behavior in the non-human animal, and wherein the abnormal emotion or behavior is selected from the group consisting of aggression, hyperactivity, destruction, abnormal sleep, abnormal feeding, abnormal drinking, abnormal grooming, abnormal elimination, socialization disorder, barking, growling, biting, mouthing, play aggression, and any combination thereof, is provided. In one embodiment, the non-human animal is a companion animal, a domestic animal, a pet, a farm animal or a pack animal. In another embodiment, the non-human animal is a dog, a cat, a goat, a pig, a sheep, a cattle, a chicken, a donkey, a llama, a camel, an elephant, a monkey, a chimpanzee, a gorilla, a deer, a duck, a goose, a pigeon, a turkey, a rabbit, a squirrel, a mouse, a rat, a hamster, a guinea pig or a horse. In accordance with the present disclosure, the method comprises administering a composition, wherein the composition comprises an effective amount of a *Lactobacillus plantarum* subsp. *plantarum* PS128, which is deposited under DSMZ Accession No. DSM 28632, and a carrier. In one embodiment of the present disclosure, the composition is orally administrated to the non-human animal.

In one embodiment, the effective amount of PS128 administered per kilogram bodyweight per day is at least 1×10⁷ CFU, at least 1×10⁸ CFU, at least 1×10⁹ CFU, at least 1×10¹⁰ CFU or at least 1×10¹¹ CFU, including 1×10⁷ CFU, 2×10⁷ CFU, 3×10⁷ CFU, 4×10⁷ CFU, 5×10⁷ CFU, 6×10⁷ CFU, 7×10⁷ CFU, 8×10⁷ CFU, 9×10⁷ CFU, 1×10⁸ CFU, 2×10⁸ CFU, 3×10⁸ CFU, 4×10⁸ CFU, 5×10⁸ CFU, 6×10⁸ CFU, 7×10⁸ CFU, 8×10⁸ CFU, 9×10⁸ CFU, 1×10⁹ CFU, 2×10⁹ CFU, 3×10⁹ CFU, 4×10⁹ CFU, 5×10⁹ CFU, 6×10⁹ CFU, 7×10⁹ CFU, 8×10⁹ CFU, 9×10⁹ CFU, 1×10¹⁰ CFU, 2×10¹⁰ CFU, 3×10¹⁰ CFU, 4×10¹⁰ CFU, 5×10¹⁰ CFU, 6×10¹⁰ CFU, 7×10¹⁰ CFU, 8×10¹⁰ CFU, 9×10¹⁰ CFU, 1×10¹¹ CFU, 2×10¹¹ CFU, 3×10¹¹ CFU, 4×10¹¹ CFU, 5×10¹¹ CFU, 6×10¹¹ CFU, 7×10¹¹ CFU, 8×10¹¹ CFU, and 9×10¹¹ CFU, but not limited thereto.

In one embodiment, the dosage of PS 128 administered per kilogram bodyweight per day is at least 0.01 g, at least 0.02 g, at least 0.03 g, at least 0.04 g or at least 0.05 g. In another embodiment, the dosage of PS128 administered per kilogram bodyweight per day is 0.01 g, 0.015 g, 0.02 g, 0.025 g, 0.03 g, 0.035 g, 0.04 g, 0.045 g or 0.05 g.

In one embodiment of the present disclosure, the abnormal emotion or behavior is aggression. In a further embodiment, the abnormal emotion or behavior includes one of hyperactivity, destruction, abnormal sleep, abnormal feeding, abnormal drinking, abnormal grooming, abnormal elimination, socialization disorder, barking, growling, biting, mouthing, play aggression, and any combination thereof as defined by the claims.

In the embodiment of the present application, a score or a value for evaluating the abnormal emotion or behavior in the non-human animal is improved. In a further embodiment, the evaluating includes evaluation of dog's emotional and cognitive disorders (EDED) or scoring of canine behavior checklist.

In the embodiment of the present disclosure, after the administration of the composition, the value of the evaluation of dog's emotional and cognitive disorders (EDED value) is statistically significantly decreased. In another embodiment, after the administration of the composition, at least a score of canine behavior checklist is statistically significantly decreased. In still another embodiment, after the administration of the composition, the total score of canine behavior checklist is statistically significantly decreased.

### BRIEF DESCRIPTION OF THE DRAWINGS

FIG. 1 shows the total scores of the canine behavior checklist before and after oral administration of PS128 (data shown as the means ± S.D., n = *20, p* < 0.001).
FIG. 2 shows the EDED value before and after oral administration of PS128 (data shown as the means ± S.D., n = 20*, p* < 0.005).

### DETAILED DESCRIPTION OF THE EMBODIMENTS

The following examples are used for illustrating the disclosure. A person skilled in the art can easily conceive the other advantages and effects of the disclosure from these examples. The disclosure can also be implemented by various modifications and changes that do not depart from the spirit of the disclosure. It is understood that the examples provided below are only exemplary of the disclosure and should not be taken as a limit to the scope of the disclosure.

### Fears and Phobias

Fear is a normal response to an actual or perceived threatening stimulus or situation. Apprehension or anxiety is a response to fear and agitation, when the animal anticipates a threat or fearful situation. Phobia is an exaggerated fear response. The fear response may include panting and salivation, tucked tail, lowered ears, gazing away, low body posture, piloerection, vocalization, or displacement behaviors such as yawning or lip licking. While avoidance and escape is one strategy, some dogs use aggression to remove the fear-evoking stimulus and are reinforced by success (negative reinforcement).

Some of the more common presentations include the following: 1) fear of other dogs, especially those that are unfamiliar, appearing threatening to the dog, or with which the dog has had an unpleasant experience; 2) fear of unfamiliar people, especially those who are novel or look, act, or smell different than those the dog is accustomed to (e.g., young children); 3) fear of inanimate stimuli such as loud or unfamiliar noises (e.g., construction work, trucks, gunshot), visual stimuli (e.g., umbrellas, hats, uniforms), environments (e.g., backyard, park, boarding kennel), surfaces (e.g., grass, tile or wood floors, steps), or a combination of stimuli (e.g., vacuum cleaners, car rides); and 4) fear of specific situations such as veterinary clinics or grooming parlors.

Phobic responses in dogs are generally associated with loud noises (e.g., thunder, fireworks, gunshots), and the stimuli associated with these events include rain, lightning, and perhaps even static or pressure changes associated with a thunderstorm. Some fears (e.g., veterinary clinics, going outdoors, entering certain rooms, or walking on certain types of flooring) may become so intense that they meet the definition of a phobia.

### Separation Anxiety

Separation anxiety is an inability of the pet to find comfort when separated from family members. The problem may be primary (e.g., hyperattachment, dysfunctional attachment) as the puppy ages and matures; in fact, the chances of the problem developing can be reduced by having puppies regularly spend time during the day on their own (e.g., in a safe haven). In other cases, the anxiety about being left alone is secondary to an event such as a change in the household or dog's daily routine, or associated with an underlying state of anxiety along with other behavioral issues such as noise phobias and separation anxiety. Anxiety may lead to destructive behavior (e.g., at exits or toward owner possessions), distress vocalization, house soiling, salivation, pacing, restlessness, inability to settle, anorexia, and repetitive or compulsive behaviors. The behaviors are exhibited when the dog is left alone, and generally arise within the first 15 to 30 min after departure. Many pets with separation anxiety begin to exhibit signs as the owner prepares to depart (e.g., putting on shoes, getting keys, or going to the door). When the owner is home, the dog may crave constant contact or proximity to the owner. When the owner returns, the welcoming responses are commonly exaggerated, and the dog is hard to calm down.

### Aggression

Aggression refers to threatening behaviors or harmful attacks and can range from subtle changes in body posture, facial expressions, and vocalization to biting, and can be fear-related aggression, possessive aggression (resource guarding), play aggression, redirected aggression, irritable/conflict/impulse control aggression, aggression toward other dogs, territorial/protective aggression, and predatory aggression.

### EXAMPLES

### EXAMPLE 1: Preparation of PS128

*Lactobacillus plantarum* subsp. *plantarum* PS128 (hereinafter referred to as PS128) used in the study was the strain isolated and deposited under DSMZ Accession No. DSM 28632. PS128 was inoculated in a culture medium (10% skim milk, 1% yeast powder, 0.1% Tween 80, and 2% glucose), cultured at 37°C for 18 hours and harvested by centrifugation. PS128 was embedded and lyophilized with protective agents (1% skim milk, 2% sugar, 2% oligofructose, 3% maltodextrin, and 2% glycerol) and excipients to a final concentration of 5×10¹⁰ colony formation unit (CFU) per gram powder.

### EXAMPLE 2. Treating abnormal emotions and behaviors with PS128

A total of 20 dogs were examined in the veterinary clinic for their abnormal emotions and behaviors. They were evaluated and given score by professional veterinarian with the canine behavior checklist as shown in Table 1 and the Evaluation of Dog's Emotional Disorders (EDED) scale as shown in Table 2 below. The checklist and the EDED scale are used by the veterinarian for evaluating abnormal emotions and behaviors in dogs (Landsberg G., Hunthausen W., Ackerman L., 2013, Behavior Problems of the Dog and Cat. Saunders, Edinburgh 2013, Elsevier Ltd.). Total scores were calculated and obtained for each dog. The tested dogs were further examined by veterinarian to exclude the abnormal behavior due to illness, such as the abnormal behavior due to abnormal thyroxine.

**Table 1. Canine behavior checklist**

| Canine behavior checklist | | | | | |
|---|---|---|---|---|---|
| Name: | | | Today's date: | | |
| Pet's name: | | Age: | Sex: M/F | Neutered: Y/N | |
| Please use the following scoring system: | | | | | |
| Scoring: 0 - never; 1 - rarely; 2 - sometimes; 3 - frequent; 4 - all the time | | | | | |
| | | | | Score | When began? |
| 1. Fear (no aggression): People / Locations / Situations / Noises | | | | | |
| | People familiar__ unfamiliar__ | | | | |
| | Animals familiar__ unfamiliar__ | | | | |
| | Car rides__ Veterinary clinic__ Surfaces__ Storms__ | | | | |
| | Fireworks__ Vacuum__ Other:_______ | | | | |
| | Describe: | | | | |
| 2. Growl / threaten / bite unfamiliar people__ | | | | | |
| | Describe: | | | | |
| 3. Growl / threaten / bite family members__ | | | | | |
| | Describe: | | | | |
| 4. Growl / threaten / bite other family pets__ | | | | | |
| | Describe: | | | | |
| 5. Growl / threaten / bite unfamiliar dogs__ | | | | | |
| | Describe: | | | | |
| 6. Mouthing / grabbing / play biting__ | | | | | |
| | Describe: | | | | |
| 7. When left alone: | | | | | |
| | anxious__ destructive__ vocal__ soils__ salivate__ | | | | |
| 8. Soiling indoors: urine__ stools__ | | | | | |
| | | | Left alone:__ Family at home__ | | |
| | | | Describe: | | |
| 9. Destructive: Chews__ Digs__ Other: | | | | | |
| 10. Steals: garbage__ food__ toys__ Other: | | | | | |
| | | | Describe: | | |
| | | | If yes, will he drop/give? Y N | | |
| | | | Is he possessive/aggressive? Y N | | |
| 11. Barking at doors, windows, fences?__ Other: | | | | | |
| | | | Describe: | | |
| 12. Excitable/won't settle__ Jumps on people__ | | | | | |
| | | | Gets on furniture__/counters | | |
| | | | Describe: | | |
| 13. Difficult to train__ Ignores commands__ Pulls on walks__ | | | | | |
| | | | Other: | | |
| | | | Describe: | | |
| 14. Repetitive behaviors - | | | | | |
| | | | Chews/licks self__ Chases tail__ Licks/suck__ Staring__ | | |
| | | | Circling__ Snaps at air__ Chases lights__ Other: | | |
| | | | Describe: | | |
| 15. Mounting other dogs__ Household objects__ Masturbate__ | | | | | |
| 16. Chases people__ animals__ cars__ bikes__ wildlife__ | | | | | |
| | | | Other: | | |
| | | | Describe: | | |
| 17. Ingestive - Eating: | | | | | |
| | | | Voracious / gulps__ Picky__ Excessive drinking__ | | |
| | Eats stool__ Eats other nonfood items (pica)__ | | | | |
| | Describe: | | | | |
| 18. Attention seek__ Clingy__ | | | | | |
| | Describe: | | | | |
| 19. Wakes at night__ | | | | | |
| 20. Other / describe: | | | | | |
| If you need assistance with any of the above - indicate # | | | | | |

**Table 2. Evaluation of dog's emotional and cognitive disorders (EDED scale)**

| The EDED scale - evaluation of dog's emotional and cognitive disorders | | | | |
|---|---|---|---|---|
| Behavior type | Specific behavior | Score | Date | Score |
| Centripetal | | | | |
| Feeding | Normal appetite | | | 1 |
| | Hyperphagia (with regurgitation and reingestion)¹ | | | 3 |
| | Anorexia/hyporexia | | | 4 |
| | Dysorexia (moving from hyper to hypo) | | | 5 |
| Drinking | Normal drinking | | | 1 |
| | Carries empty water bowl around (ritual)² | | | 2 |
| | Chews at water without swallowing³ | | | 3 |
| | High-frequency drinking (documented) | | | 5 |
| Self-stimulatory | Normal cleaning behavior | | | 1 |
| | Excessive licking, nibbling⁴ | | | 4 |
| | Stereotyped nibbling, dizziness, turning on itself (or other stereotypies)⁵ | | | 5 |
| Sleep | Normal (or no change) | | | 1 |
| | Increase in sleep, hypersomnia⁶ | | | 2 |
| | Insomnia, during sleep (and hyposomnia)⁷ | | | 3 |
| | Wakes up shortly after falling asleep, anxiety at time of going to sleep (and restlessness)⁸ | | | 5 |

| Centrifugal | | | | |
|---|---|---|---|---|
| Exploratory (scanning) | Normal | | | 1 |
| | Inhibited | | | 2 |
| | Frequent avoidance responses | | | 3 |
| | Increased, hypervigilant | | | 4 |
| | Oral | | | 5 |
| Aggression (defense) | No aggression or aggression stable (no increase or decrease) | | | 1 |
| | Irritation-related aggression | | | 3 |
| | Fear-related aggression | | | 4 |
| | Displays both fear and irritation aggression | | | 5 |
| Learned social behavior | Unchanged | | | 1 |
| | No submission response | | | 2 |
| | No self-control when playing | | | 2 |
| | Bites without growling | | | 4 |
| | Steals, does not drop stolen objects | | | 5 |
| Specific learned behavior | Same response capacity (allowing for disease or age) | | | 1 |
| | Arbitrary responses | | | 3 |
| | No response to previously learned behaviors | | | 5 |
| Physical exam⁹ | Normal | | | 1 |
| | Periods of tachycardia and/or tachypnea | | | 2 |
| | Diarrhea, colic | | | 2 |
| | Dyspepsia (and ptyalism) | | | 2 |
| | Increased emotional micturition | | | 3 |
| | Acral lick granuloma (and extensive lick alopecia) | | | 4 |
| | Obesity | | | 4 |
| | High-quantity drinking and urination (PU/PD) | | | 4 |
| Total | | | | |
| Centripetal, internal factors; centrifugal, external stimuli; PU, polyuria; PD, polydipsia. | | | | |
| ¹ Hyperphagia with regurgitation and reingestion. The dog eats rapidly, displays spasms, followed by vomiting. It then reingests what it has just expelled and resumes its meal. This behavior appears regularly (1 meal in 2). | | | | |
| ² Carries empty water bowl around. The dog moves or carries its bowl toward one or several family members. This behavior stops as soon as the bowl is filled. | | | | |
| ³ Chews at water without swallowing it (dipsomania). The dog nibbles the water and spreads it around its bowl while swallowing very little. | | | | |
| ⁴ Excessive licking, nibbling. A dog that is licking or nibbling itself, then spontaneously stops. | | | | |
| ⁵ Stereotypic nibbling, dizziness. When there is licking or nibbling that does not stop spontaneously (the owners must stop the dog or divert its attention) or else dizziness or any other stereotypy (e.g., licking of the face, jumping, wandering). | | | | |
| ⁶ Increase in sleep, hypersomnia. When the duration of sleep is longer than age norm (+25%). the | | | | |
| ⁷ Insomnia, during sleep. Awakenings appear more than 90 minutes after going to sleep. | | | | |
| ⁸ Wakes up shortly after going to sleep, anxiety at time of going to sleep. dog awakes in the 30-45 minutes following going to sleep. Prior to going sleep the dog may display moans, excitement, and a search for contacts, as is afraid of going to sleep. toif Theit | | | | |
| ⁹ In order to take the physical examination into account, all the manifestations observed must be scored and counted. | | | | |

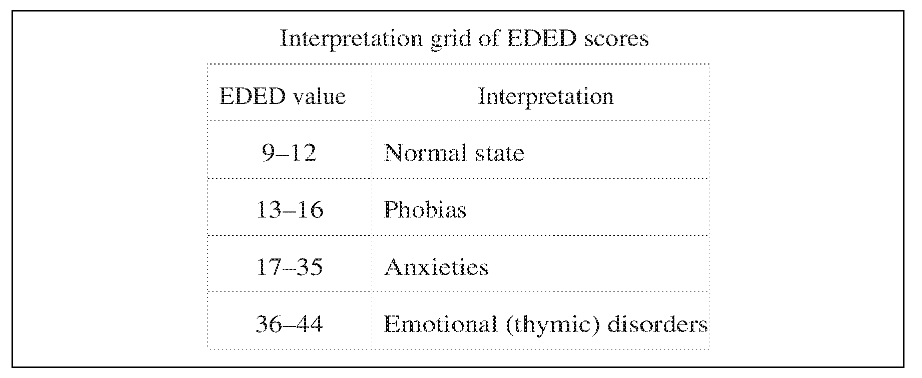

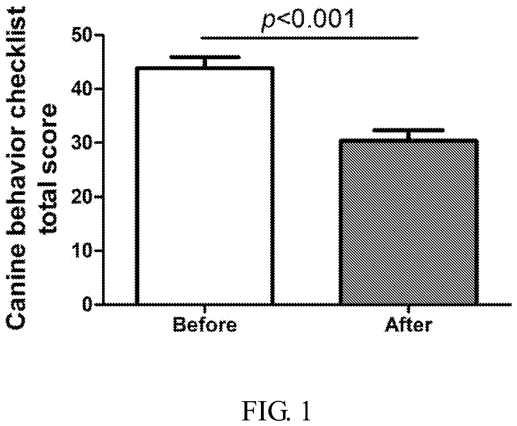

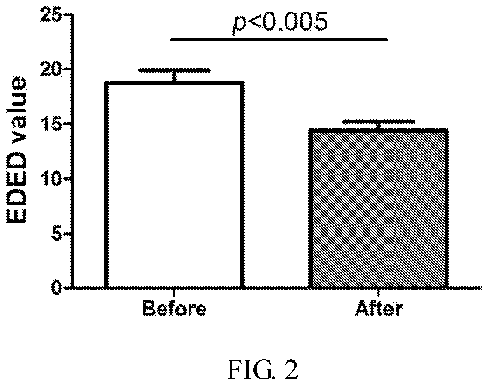

After the dogs were evaluated in the veterinary clinic for their abnormal emotions and behaviors, they were given PS128 at a dosage of 0.02 g/kg bodyweight/day for a period of 2 weeks. After administration of PS128 for 2 weeks, they were evaluated again for their abnormal emotions and behaviors with the same behavior checklist and EDED scale. The results of the behavior checklist were summarized in FIG. 1, and the results of the EDED scale were illustrated in FIG. 2. The total score of the evaluation before and after administration of PS128 were recorded and analyzed.

As shown in FIGs. 1 and 2, the total scores of both the canine behavior checklist and the EDED scale decreased significantly after PS128 administration compared to those before the PS128 administration, indicating the effect of PS128 in preventing and treating abnormal emotions and behaviors in these companion animals.

While some of the embodiments of the present disclosure have been described in detail in the above, it is, however, possible for those of ordinary skill in the art to make various modifications and changes to the embodiments shown without departing from the scope of the claims. Such modifications and changes are encompassed in the scope of the appended claims.

## Claims

1. A composition for use in preventing or treating an abnormal emotion or behavior in a non-human animal, **characterized in that** the composition comprises an effective amount of *Lactobacillus plantarum* subsp. *plantarum* PS128, which is deposited under DSMZ Accession No. DSM 28632, and a carrier thereof, wherein the composition improves a score or a value for evaluating the abnormal emotion or behavior in the non-human animal, and wherein the abnormal emotion or behavior is selected from the group consisting of aggression, hyperactivity, destruction, abnormal sleep, abnormal feeding, abnormal drinking, abnormal grooming, abnormal elimination, socialization disorder, barking, growling, biting, mouthing, play aggression, and any combination thereof.

2. The composition for use as claimed in claim 1, wherein the non-human animal is a companion animal, a domestic animal, a pet, a farm animal or a pack animal.

3. The composition for use as claimed in claim 1, wherein the non-human animal is selected from the group consisting of a dog, a cat, a goat, a pig, a sheep, a cattle, a chicken, a donkey, a llama, a camel, an elephant, a monkey, a chimpanzee, a gorilla, a deer, a duck, a goose, a pigeon, a turkey, a rabbit, a squirrel, a mouse, a rat, a hamster, a guinea pig and a horse.

4. The composition for use as claimed in claim 1, wherein the composition is orally administrated to the non-human animal in an amount of at least 10⁷ CFU of the *Lactobacillus plantarum* subsp. *plantarum* PS128 per day.

5. The composition for use as claimed in claim 1, wherein the composition is orally administered to the non-human animal at a dosage of at least 0.01 g of the *Lactobacillus plantarum* subsp. *plantarum* PS 128/kg bodyweight/day.

6. The composition for use as claimed in claim 1, wherein the composition is administered to the non-human for at least one week.

7. The composition for use as claimed in claim 1, wherein the evaluating includes evaluation of dog's emotional and cognitive disorders (EDED), and an EDED value in the non-human animal is decreased.

8. The composition for use as claimed in claim 1, wherein the evaluating includes scoring of canine behavior checklist, and at least a score of the canine behavior checklist in the non-human animal is decreased.

## Patentansprüche

1. Zusammensetzung zur Verwendung bei der Vorbeugung oder Behandlung eines abnormalen Gefühls oder Verhaltens bei einem nicht-menschlichen Tier, **dadurch gekennzeichnet, dass** die Zusammensetzung eine wirksame Menge an *Lactobacillus plantarum* subsp. *plantarum* PS128, die unter der DSMZ-Zugangsnummer DSM 28632 hinterlegt ist, und einen Träger davon umfasst, wobei die Zusammensetzung eine Punktzahl oder einen Wert zur Bewertung des abnormalen Gefühls oder Verhaltens bei dem nicht-menschlichen Tier verbessert und wobei das abnormale Gefühl oder Verhalten aus der Gruppe ausgewählt ist, die aus Aggression, Hyperaktivität, Zerstörungswut, abnormalem Schlafverhalten, abnormalem Fressverhalten, abnormalem Trinkverhalten, abnormaler Fellpflege, abnormaler Ausscheidung, Sozialisationsstörungen, Bellen, Knurren, Beißen, Kauen, spielerischer Aggression und jeder Kombination davon besteht.

2. Zusammensetzung zur Verwendung nach Anspruch **1,** wobei das nicht-menschliche Tier ein Begleittier, ein Heimtier, ein Haustier, ein Nutztier oder ein Lasttier ist.

3. Zusammensetzung zur Verwendung nach Anspruch 1, wobei das nicht-menschliche Tier aus der Gruppe ausgewählt ist, die aus einem Hund, einer Katze, einer Ziege, einem Schwein, einem Schaf, einem Rind, einem Huhn, einem Esel, einem Lama, einem Kamel, einem Elefanten, einem Affen, einem Schimpansen, einem Gorilla, einem Reh, einer Ente, einer Gans, einer Taube, einem Truthahn, einem Kaninchen, einem Eichhörnchen, einer Maus, einer Ratte, einem Hamster, einem Meerschweinchen und einem Pferd besteht.

4. Zusammensetzung zur Verwendung nach Anspruch 1, wobei die Zusammensetzung dem nicht-menschlichen Tier oral in einer Menge von mindestens 10⁷ KBE *Lactobacillus plantarum* subsp. *plantarum* PS128 pro Tag verabreicht wird.

5. Zusammensetzung zur Verwendung nach Anspruch 1, wobei die Zusammensetzung dem nicht-menschlichen Tier oral in einer Dosierung von mindestens 0,01 *g Lactobacillus plantarum* subsp. *plantarum* PS128 / kg Körpergewicht / Tag verabreicht wird.

6. Zusammensetzung zur Verwendung nach Anspruch 1, wobei die Zusammensetzung dem nicht-menschlichen Tier mindestens eine Woche lang verabreicht wird.

7. Zusammensetzung zur Verwendung nach Anspruch 1, wobei das Bewerten die Bewertung der emotionalen und kognitiven Störungen (EDED) des Hundes beinhaltet und ein EDED-Wert bei dem nicht-menschlichen Tier verringert ist.

8. Zusammensetzung zur Verwendung nach Anspruch 1, wobei das Bewerten die Vergabe einer Punktzahl in einer Checkliste für das Verhalten von Hunden beinhaltet und mindestens eine Punktzahl in der Checkliste für das Verhalten von Hunden bei dem nicht-menschlichen Tier verringert ist.

## Revendications

1. Composition destinée à être utilisée dans la prévention ou le traitement d'une émotion ou d'un comportement anormal chez un animal non humain, **caractérisée en ce que** la composition comprend une quantité efficace de *Lactobacillus plantarum* subsp. *plantarum* PS128, qui est déposée sous DSMZ n° d'accès DSM 28632 et un de ses véhicules, la composition améliorant un score ou une valeur pour évaluer l'émotion ou le comportement anormal chez l'animal non humain et l'émotion ou le comportement anormal étant choisi dans le groupe constitué par l'agression, l'hyperactivité, la destruction, le sommeil anormal, l'alimentation anormale, la boisson anormale, la toilette anormale, l'élimination anormale, le trouble de la socialisation, l'aboiement, le grognement, la morsure, la mise en bouche, l'agression par jeu et l'une quelconque de leurs combinaisons.

2. Composition destinée à être utilisée selon la revendication 1, dans laquelle l'animal non humain est un animal de compagnie, un animal domestique, un animal apprivoisé, un animal de ferme ou une bête de somme.

3. Composition destinée à être utilisée selon la revendication 1, dans laquelle l'animal non humain est choisi dans le groupe constitué par un chien, un chat, une chèvre, un cochon, un mouton, un bœuf, un poulet, un âne, un lama, un chameau, un éléphant, un singe, un chimpanzé, un gorille, un cerf, un canard, une oie, un pigeon, une dinde, un lapin, un écureuil, une souris, un rat, un hamster, un cochon d'inde et un cheval.

4. Composition destinée à être utilisée selon la revendication 1, dans laquelle la composition est administrée oralement à l'animal non humain en une quantité d'au moins 10⁷ CFU de *Lactobacillus plantarum* subsp. *plantarum* PS128 par jour.

5. Composition destinée à être utilisée selon la revendication 1, dans laquelle la composition est administrée oralement à l'animal non humain à une dose d'au moins 0,01 g de *Lactobacillus plantarum* subsp. *plantarum* PS128/kg de poids corporel/jour.

6. Composition destinée à être utilisée selon la revendication 1, dans laquelle la composition est administrée à l'animal non humain durant au moins une semaine.

7. Composition destinée à être utilisée selon la revendication 1, dans laquelle l'évaluation comprend l'évaluation de troubles émotionnels et cognitifs d'un chien (EDED) et une valeur d'EDED chez l'animal non humain est diminuée.

8. Composition destinée à être utilisée selon la revendication 1, dans laquelle l'évaluation comprend la notation de la liste de vérification de comportement canin et au moins un score de la liste de vérification de comportement canin chez l'animal non humain est diminuée.
